# EUROPÄISCHE PATENTSCHRIFT

(11) **EP 2 420 318 B1**
(45) Veröffentlichungstag und Bekanntmachung des Hinweises auf die Patenterteilung: **10.09.2014**
(21) Anmeldenummer: 10174561.0
(22) Anmeldetag: 30.08.2010
(51) Int. Cl.: B01L 3/00

(54) **Tupfer zur Erfassung und sterilen Aufbewahrung von biologischen Proben**
Swab for taking biological samples and storing them in a sterile environment
Coton destiné à la détection et au stockage stérile d'échantillons biologiques

(30) Priorität: 14.07.2010 DE 202010010203 U
(43) Veröffentlichungstag der Anmeldung: 22.02.2012
(73) Patentinhaber: Helling GmbH, 25436 Heidgraben (DE)
(72) Erfinder: Riess, Nathanael, 25436 Uetersen (DE)
(74) Vertreter: Meyer, Ludgerus

(56) Entgegenhaltungen:
- WO-A1-93/11434
- US-A1- 2005 009 200
- US-A1- 2008 003 144

## Beschreibung

Die Erfindung betrifft einen Tupfer zur Erfassung und sterilen Aufbewahrung von biologischen Proben, welche DNA-Material enthalten, nach dem Oberbegriff des Anspruchs 1.

Zur Spurensicherung an Tatorten werden heute zunehmend DNA-Proben genommen, die mit hoher Sicherheit eine Zuordnung der Probe zu einem Täter ermöglichen, wobei zur DNA-Analyse lediglich wenige Körperzellen ausreichend sind. Wenn eine geringe Zahl von Zellen vorhanden ist, ist es aber wichtig, dass die vorhandenen Spuren nicht noch durch Verunreinigungen oder Fremd-DNA-Spuren beeinträchtigt werden.

Eine große Schwachstelle bei einer forensischen Untersuchung sind offenbar die Testmaterialien selbst. So wurde ein Fall bekannt, in dem mit Fremd-DNA verunreinigte Wattestäbchen benutzt wurden, die die Ermittler auf eine falsche Fährte schickten. Obgleich die Herstellung derartiger Wattestäbchen in der Regel unter reinraumähnlichen Bedingungen steril erfolgt, sind sie zwar frei von Mikroorganismen, jedoch kann gleichwohl Fremd-DNA-Material am Wattestäbchen vorhanden sein, das auch bei einer DNA-Aufnahme leicht an das Wattestäbchen gelangen kann, wenn die Aufnahme der Probe unter erschwerten Bedingungen eines Tatorts zu erfolgen hat.

Es ist bekannt, Wattestäbchen durch radioaktive Bestrahlung steril zu machen. Es ist auch bekannt, die Produktion von Wattestäbchen dadurch zu verbessern, dass diese mit Ethylenoxid begast werden, wodurch DNA-Spuren deaktiviert werden. Die Begasung mit Ethylenoxid hat jedoch auch den Nachteil, dass später noch vorhandene Ethylenoxidgase die zu erfassenden DNA-Spuren zerstören können.

Aus der DE 199 57 861 A1 ist ein Probenbehälter zur Lagerung und Identifizierung von DNA/RNA-haltigen Material bekannt, bei dem der Probenbehälter eine starke hygroskopische Substanz enthält. Diese besteht z. B. aus Zeolith, Silikagel oder einem Molekularsieb, welches in einem getrennten Teil des Probenbehälters angeordnet ist. Durch die Aufteilung des Probenbehälters in einen Proberaum und mindestens einen Raum für die hygroskopische Substanz können Probe und hygroskopische Substanz getrennt verwendet werden. Die Verwendung der hygroskopischen Substanzen kann die Stabilität der zu analysierenden Probe verbessern. Das Problem der Verschmutzung des Probenbehälters selbst durch Fremd-DNA ist in dieser Druckschrift jedoch nicht angesprochen.

Aus der DE 60 2004 010 240 T2 ist ein Tupfer zur Aufnahme von biologischen Proben bekannt, der in einem Teströhrchen aufgenommen ist, wobei der Tupfer als Tupferstab ausgebildet ist, der an seiner Spitze einen Faserbausch aus Fasern mit hydrophilen Eigenschaften aufweist, um die Absorption von Proben zu ermöglichen, wobei die Faser an der Tupferspitze in der Form einer von durch Flockung abgelagerten Schicht hergestellt ist. Auch hier ist das Problem der Erfassung von Fremd-DNA vernachlässigt.

Der Erfindung liegt die Aufgabe zugrunde, einen Tupfer zur Erfassung und sterilen Aufbewahrung von biologischen Proben, welche DNA-Material enthalten, anzugeben, bei dem die Gefahr einer Kontaminierung mit Fremd-DNA stark verringert ist, bei dem die Aufnahmefläche bis zur Aufnahme der Probe so lange wie möglich geschützt verbleiben kann, der einfach handhabbar ist und kostengünstig herstellbar ist.

Diese Aufgabe wird durch die im Anspruch 1 angegebene Erfindung gelöst. Vorteilhafte Weiterbildungen der Erfindung sind in Unteransprüchen angegeben.

Die Erfindung geht aus von einem Tupfer zur Erfassung und sterilen Aufbewahrung von biologischen Proben, welche DNA-Material enthalten, wobei der Tupfer eine an einem Träger befindlichen Aufnahmefläche zur Erfassung der Probe enthält, der nach der Erfassung der Probe in einen verschließbaren Behälter einsetzbar ist.

Erfindungsgemäß ist der Träger als Schaumstoffzylinder ausgebildet, dessen erstes Ende in einen Stopfen eingesetzt ist, der auf den einseitig offenen röhrchenförmig ausgebildeten Behälter gasdicht aufsetzbar ist. Das zweite Ende des Schaumstoffzylinders ist mit einer durch eine Klebefolie am Träger befestigten Aufnahmefläche zur Aufnahme der Probe versehen, wobei die Aufnahmefläche im unbenutzten Zustand des Tupfers mit einer Schutzfolie abgedeckt ist.

Der erfindungsgemäße Tupfer enthält daher eine Aufnahmefläche, die nicht nur in einem Röhrchen gasdicht aufgenommen ist, sondern die auch selbst innerhalb des Röhrchens abgedeckt ist, so dass die Aufnahmefläche solange geschützt bleiben kann, bis die DNA-Aufnahme erfolgt. Damit ergibt sich eine doppelte Sicherheit gegen Fremd-DNA-Einflüsse. Der verwendete Schaumstoffzylinder erlaubt es, eine Probe durch flexibles Andrücken auf eine Fläche aufzunehmen, wobei die Aufnahmefläche durch die Flexibilität des Schaumstoffs der Oberflächenkontur der abzutastenden Fläche folgen kann. Die verwendete Schutzfolie kann nach Entnahme des Probenhalters aus dem Aufnahmeröhrchen noch so lange auf der Aufnahmefläche verbleiben, bis die Aufnahme der DNA-Probe selbst erfolgen soll. Damit ist höchste Sicherheit gegen Kontamination gewährleistet. Um die Probe auch nach der Aufnahme der DNA weiter gegen Fremdeinflüsse zu sichern, kann die Abdeckfolie erneut auf die Aufnahmefläche aufgebracht werden, von der sie erst wieder im Labor kurz vor der Auswertung der Probe entfernt wird.

Der erfindungsgemäße Tupfer ist vorzugsweise zylindrisch mit einem Griffende und einem Halteende gestaltet, wobei das Griffende abdichtend auf das offene Ende des Behälters aufsetzbar ist. Das Halteende ist hohlzylindrisch derart ausgebildet, dass der Träger unter Klemmkraft in das Halteende einsetzbar ist. Zur Erhöhung der Haltekraft des Trägers am Halteende kann der Träger auch in dem Halteende festgeklebt sein.

In weiterer Ausgestaltung kann die Schutzfolie eine silikonisierte Papierabdeckung sein, die durch Haftklebung mehrfach auf die Aufnahmefläche aufgelegt werden kann.

Die Aufnahmefläche besteht insbesondere aus doppelseitig klebenden Kunststoffpunkten, deren Klebkraft gegenüber dem Träger erheblich höher als gegenüber der silikonisierten Abdeckung ist. Damit wird vermieden, dass beim Abziehen der Abdeckung der Kunststoffpunkt vom Träger abgelöst wird.

Der röhrchenförmige Behälter zur Aufnahme des Tupfers ist insbesondere als durchsichtiges Röhrchen ausgebildet. Auf der Außenseite des Röhrchens können sich Beschriftungsfelder befinden, die der Identifizierung der erfassten Probe dienen können.

Die Aufnahmefläche kann insgesamt eben sein oder auch dachförmig mit zwei Aufnahmeflächen, die z. B. zwei Proben aufnehmen können.

In einer weiterbildenden Ausführungsform kann auch vorgesehen sein, den Träger bzw. den Stopfen mit einem integrierten RFID-Transponder zu versehen, um ggf. eine automatisierte Auswertung von Proben zu ermöglichen, durch die verhindert werden kann, dass im Labor eine manuelle Behandlung der mit DNA-Spuren versehenen Probe erforderlich ist.

Zur Herstellung des Tupfers mit steriler Aufnahmefläche zum Empfang von DNA-Material ist insbesondere vorgesehen, dass der Tupfer vor seiner Benutzung in einer Behandlungskammer sterilisiert und entgast wird. Die Sterilisierung erfolgt insbesondere durch Ethylenoxid, wobei der Tupfer nach der Sterilisierung so lange entgast wird, bis der Restwert des Gases den Wert 1 ppm unterschritten hat. Insbesondere erfolgt die Entgasung während eines Zeitraums von wenigstens 20, insbesondere 30 Tagen.

Auf diese Weise wird jegliches DNA-Fremdmaterial, das bei der Herstellung des Tupfers u. U. auf die Aufnahmefläche gelangt sein kann, zerstört, ohne dass nach Ablauf der Entgasungszeit noch Sterilisationsgas vorhanden ist, das eine neue aufzunehmende Probe zerstören könnte.

Die Erfindung wird nachstehend anhand eines Ausführungsbeispiels näher erläutert. Es zeigen:
- Fig. 1: einen Querschnitt durch einen erfindungsgemäßen Tupfer, der in ein Röhrchen eingesetzt ist, und
- Fig. 2: eine vergrößerte Ansicht der Elemente der Aufnahmefläche.

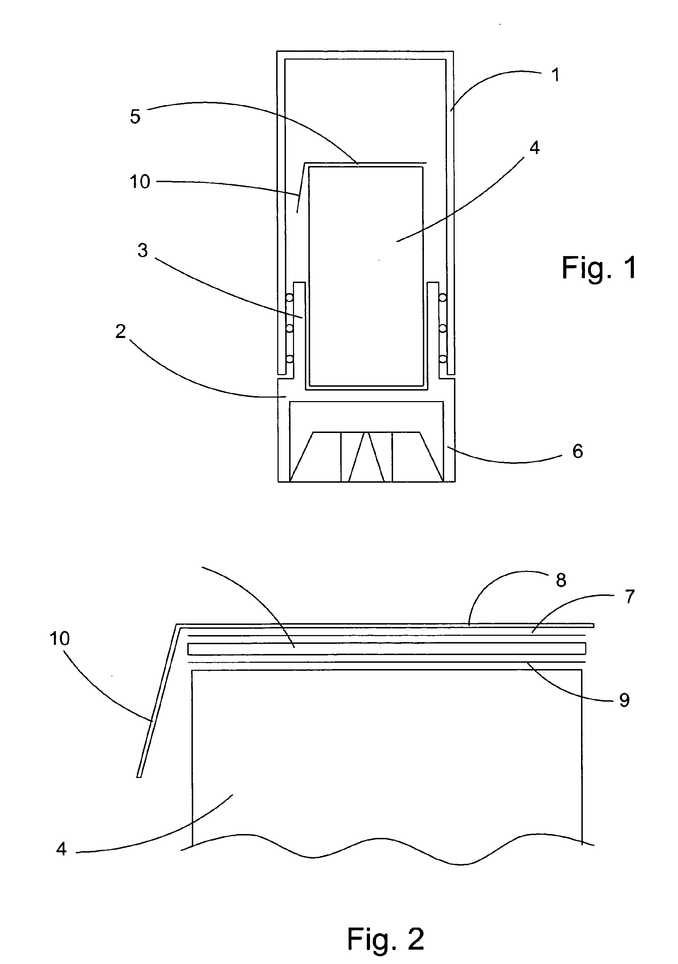

Fig. 1 zeigt ein hohlzylindrisches Glas- oder Kunststoffröhrchen 1, insbesondere aus Polystyrol, in das der aus Kunststoff, wie PVC oder Polyamid, vorzugsweise Polyethylen, hergestellte Stopfen 2 einsetzbar ist. Dieser weist auf seinem Stopfenrand umlaufende ringförmige Erhebungen zur Abdichtung gegenüber dem Behälter 1 auf.

Der Stopfen 2 enthält ein hinteres Griffende und eine vorderes Halteende 3, welches eine zylinderförmige Ausnehmung aufweist, in die ein weichelastischer Schaumstoffzylinder 4, vorzugsweise aus vernetztem LDPE einsetzbar ist. Dieser ist in dem Halteende 3 entweder eingeklemmt oder darin eingeklebt.

Am vorderen Ende des Schaumstoffzylinders 4 befindet sich eine auf die Stirnseite des Schaumstoffzylinders aufgeklebte Aufnahmefläche, deren Rückseite gegenüber dem Schaumstoffzylinder eine hohe Klebekraft aufweist und deren Vorderseite eine etwa um den Faktor 20 geringere Klebekraft aufweist. Auf die offene Vorderseite ist eine Schutzfolie 8 aufgesetzt, die an der Aufnahmefläche nur haftet und daher leicht von der Aufnahmefläche ablösbar ist. Zum Entfernen der Schutzfolie 8 weist diese einen seitlichen Anfassstreifen 10 auf, mit dessen Hilfe die Schutzfolie 8 von dem Schaumstoffkörper 4 entfernt werden kann.

Fig. 2 zeigt den Klebebereich mit Schaumstoffzylinder 4 und Aufnahmefläche 5 in vergrößerter Darstellung. Die Aufnahmefläche 5 weist auf ihrer Rückseite eine dünne Klebeschicht 9 und auf ihrer Vorderseite eine dünne Klebeschicht 7 auf. Die Aufnahmefläche 5 besteht insbesondere aus Polyesterfolie, die mit einem modifizierten Lösemittelacrylat am Schaumstoffkörper 4 festgeklebt ist. Die Klebeschicht 7 auf der Vorderseite ist ein Dispersionsreinacrylat, das schwach haftend ist und mit einer aus Silikonpapier bestehenden Schutzfolie 8 abgedeckt wird. Die Schwerfestigkeiten zwischen der Klebeschicht 7 und der Klebeschicht 9 unterscheiden sich im Verhältnis von etwa 1:20.

Die Herstellung des erfindungsgemäßen Tupfers erfolgt dadurch, dass der Schaumstoffkörper 4 zunächst in dem Halteende 3 befestigt wird, dass danach die Aufnahmefläche 5 zusammen mit der Schutzfolie 8 auf die Stirnseite des Schaumstoffkörpers 4 aufgebracht wird und der Stopfen mit eingesetztem Schaumstoffkörper 4 in das Röhrchen 1 eingesetzt wird. Mehrere derart hergestellter Röhrchen werden dann in eine Sterilisationsfolie gegeben, mittels Ethylenoxid sterilisiert, welches den gesamten Tupfer durchdringt, und danach so lange entgast, bis ein minimaler Restwert von 1 ppm Ethylenoxid erreicht ist. Dieser Wert ergibt sich nach etwa 30 Tage Entgasungszeit. Danach können die Röhrchen für längere Zeit aufbewahrt werden, bis sie benötigt werden.

Zur Aufnahme einer Probe wird ein Röhrchen der Sterilisationsfolie entnommen und unmittelbar am Ort der Probe geöffnet. Dann wird die Schutzfolie 8 von der Aufnahmefläche 5 abgezogen und die auf der Vorderseite des Schaumstoffkörpers angebrachte Aufnahmefläche 5 wird auf die Stelle aufgedrückt, von der die Probe zu nehmen ist. Die Abdeckung kann dann erneut auf den Klebepunkt aufgesetzt werden. Da hierbei jedoch die Gefahr einer Fremdkombination besteht, wird die Abdeckfolie vorzugsweise weggeworfen und der Stopfen 2 mit dem Schaumstoffkörper 4 unmittelbar in das Röhrchen 1 eingeführt. Das Röhrchen wird dann einem Labor zur Untersuchung zugeleitet.

### Bezugszeichen

- 1: Röhrchen
- 2: Stopfen
- 3: Halteende
- 4: Schaumstoffzylinder, Träger
- 5: Aufnahmefläche
- 6: Griffende
- 7: Klebeschicht
- 8: Schutzfolie
- 9: Klebeschicht
- 10: Anfassstreifen

## Patentansprüche

1. Tupfer zur Erfassung und sterilen Aufbewahrung von biologischen Proben, welche DNA-Material enthalten, wobei der Tupfer eine an einem Träger (4) befestigte Aufnahmefläche (5) zur Erfassung der Probe enthält, und der Träger (4) nach Erfassung der Probe in einen verschließbaren Behälter (1) einsetzbar ist, **dadurch gekennzeichnet, dass** der Träger (4) als Schaumstoffzylinder ausgebildet ist, dessen erstes Ende in einen Stopfen (2) eingesetzt ist, der auf den einseitig offenen röhrchenförmig ausgebildeten Behälter (1) gasdicht aufsetzbar ist, und dass das zweite Ende des Schaumstoffzylinders stirnseitig mit einer mittels Klebefolie an dem Schaumstoffzylinder befestigten Aufnahmefläche (5) zur Aufnahme der Probe versehen ist, wobei die Aufnahmefläche (5) im unbenutzten Zustand des Tupfers mit einer Schutzfolie abgedeckt ist.

2. Tupfer nach Anspruch 1, **dadurch gekennzeichnet, dass** der Stopfen (2) zylindrisch mit einem Griffende (6) und einem Halteende (3) ausgestattet ist, wobei das Griffende (6) abdichtend auf das offene Ende des Behälters (1) aufsetzbar ist und das Halteende (3) hohlzylindrisch derart ausgebildet ist, dass der Träger (4) unter Klemm- oder Klebekraft in das Halteende (3) einsetzbar ist.

3. Tupfer nach Anspruch 1, **dadurch gekennzeichnet, dass** die Schutzfolie (8) eine silikonisierte Papierabdeckung mit einem Anfassstreifen (10) ist.

4. Tupfer nach Anspruch 1, **dadurch gekennzeichnet, dass** der Stopfen (2) aus Kunststoff gebildet ist.

5. Tupfer nach Anspruch 1, **dadurch gekennzeichnet, dass** die Stirnseite des mit der Klebefolie versehenen Trägers (4) rechtwinklig zur Achse des Trägers verläuft und eben ausgebildet ist.

6. Tupfer nach Anspruch 1, **dadurch gekennzeichnet, dass** die Stirnseite des Trägers (4) dachförmig mit zwei im Winkel zueinanderstehenden Flächen als Aufnahmeflächen ausgebildet ist.

## Claims

1. Swab for the collection and sterile storage of biological samples which contain DNA material, wherein the swab contains a receiving surface (5) for collecting the sample, which surface is fastened to a carrier (4), and said carrier (4) can be inserted in a closable container (1) after the collection of the sample,
**characterised in that**
the carrier (4) is designed as a foam cylinder, the first end of which is inserted in a stopper (2) which can be placed, in a gastight manner, on the container (1) which is open at one end and of tubular design, and that the second end of the foam cylinder is provided, at the end face, with a receiving surface (5) for receiving the sample, which surface is fastened to the foam cylinder by means of adhesive film, said receiving surface (5) being covered with a protective film when the swab is in the unused condition.

2. Swab according to Claim 1,
**characterised in that**
the stopper (2) is equipped cylindrically with a handle end (6) and a holding end (3), it being possible to place the handle end (6) on the open end of the container (1) in a sealing manner, and the holding end (3) being of hollow-cylindrical design in such a way that the carrier (4) can be inserted in said holding end (3) under clamping or adhesive force.

3. Swab according to Claim 1,
**characterised in that**
the protective film (8) is a siliconised paper cover with a grab strip (10).

4. Swab according to Claim 1,
**characterised in that**
the plug (2) is formed from plastic.

5. Swab according to Claim 1,
**characterised in that**
the end face of the carrier (4) provided with adhesive film extends at right angles to the axis of said carrier and is of flat design.

6. Swab according to Claim 1,
**characterised in that**
the end face of the carrier (4) is of roof-shaped design with two surfaces that are at an angle to one another as the receiving surfaces.

## Revendications

1. Tampon pour le prélèvement et le stockage stérile d'échantillons biologiques qui contiennent un matériel ADN, étant précisé que le tampon contient une surface de réception (5), fixée à un support (4), qui est destinée à prélever l'échantillon, et que le support (4), après le prélèvement de l'échantillon, est apte à être placé dans un récipient apte à être fermé (1), **caractérisé en ce que** le support (4) est conçu comme un cylindre en mousse dont une première extrémité est placée dans un bouchon (2) apte à être posé de manière étanche au gaz sur le récipient en forme de tube (1) ouvert d'un côté, et **en ce que** la seconde extrémité du cylindre en mousse est pourvue, côté frontal, d'une surface de réception (5), fixée au cylindre en mousse à l'aide d'un film adhésif, pour recevoir l'échantillon, étant précisé que la surface de réception (5), dans l'état non utilisé du tampon, est couverte par un film de protection.

2. Tampon selon la revendication 1, **caractérisé en ce que** le bouchon (2), de forme cylindrique, est pourvu d'une extrémité formant poignée (6) et d'une extrémité de fixation (3), étant précisé que l'extrémité formant poignée (6) est apte à être posée de manière étanche sur l'extrémité ouverte du récipient (1) et que l'extrémité de fixation (3), de forme cylindrique creuse, est conçue pour que le support (4) puisse être placé avec une force de serrage ou d'adhérence dans l'extrémité de fixation (3).

3. Tampon selon la revendication 1, **caractérisé en ce que** le film de protection (8) est constitué par un recouvrement en papier siliconé avec une bande de préhension (10).

4. Tampon selon la revendication 1, **caractérisé en ce que** le bouchon (2) se compose de matière plastique.

5. Tampon selon la revendication 1, **caractérisé en ce que** le côté frontal du support (4) pourvu du film adhésif s'étend à angle droit par rapport à l'axe dudit support et a une forme plane.

6. Tampon selon la revendication 1, **caractérisé en ce que** le côté frontal du support (4) a la forme d'un toit, avec comme surfaces de réception deux surfaces qui forment un angle.
